(19) Europäisches Patentamt

European Patent Office

Office européen des brevets

(11) **EP 3 328 433 B1**

(12) **EUROPEAN PATENT SPECIFICATION**

(45) Date of publication and mention of the grant of the patent:
**22.05.2019 Bulletin 2019/21**

(21) Application number: **16741762.5**

(22) Date of filing: **08.07.2016**

(51) Int Cl.:
*A61K 47/12* (2006.01)     *A61K 47/20* (2006.01)
*A61K 47/38* (2006.01)     *A61K 9/48* (2006.01)
*B29L 31/00* (2006.01)     *B29C 39/04* (2006.01)
*B29C 39/38* (2006.01)     *B29K 1/00* (2006.01)
*C08L 1/14* (2006.01)

(86) International application number:
**PCT/US2016/041460**

(87) International publication number:
**WO 2017/019277 (02.02.2017 Gazette 2017/05)**

(54) **DISPERSION COMPRISING AN ESTERIFIED CELLULOSE ETHER**

DISPERSION MIT EINEM VERESTERTEN CELLULOSEETHER

DISPERSION COMPRENANT UN ÉTHER DE CELLULOSE ESTÉRIFIÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.07.2015 US 201562197607 P**

(43) Date of publication of application:
**06.06.2018 Bulletin 2018/23**

(73) Proprietor: **Dow Global Technologies LLC Midland, MI 48674 (US)**

(72) Inventors:
• **KNARR, Matthias**
  **29699 Bomlitz (DE)**
• **FETNER, Neal J.**
  **Midland, MI 48642 (US)**

• **MALOTKY, David L.**
  **Midland, MI 48674 (US)**
• **ZHAO, Jin**
  **Midland, MI 48674 (US)**
• **BRACKHAGEN, Meinolf**
  **29699 Bomlitz (DE)**
• **PETERMANN, Oliver**
  **29699 Bomlitz (DE)**
• **ADDEN, Roland**
  **29699 Bomlitz (DE)**

(74) Representative: **f & e patent**
  **Braunsberger Feld 29**
  **51429 Bergisch Gladbach (DE)**

(56) References cited:
  **EP-A1- 0 648 487     US-B1- 7 138 143**

**Description**

FIELD

This invention concerns aqueous compositions comprising dispersed esterified cellulose ethers, processes for producing the compositions, and coated dosage forms and capsule shells made from the aqueous compositions.

INTRODUCTION

Esters of cellulose ethers, their uses and processes for preparing them are generally known in the art. Known methods of producing cellulose ether-esters include the reaction of a cellulose ether with an aliphatic monocarboxylic acid anhydride or a dicarboxylic acid anhydride or a combination thereof, for example as described in U.S Patent Nos. 4,226,981 and 4,365,060.

Various known esterified cellulose ethers are useful as enteric polymers for pharmaceutical dosage forms, such as methylcellulose phthalate (MCP), hydroxypropyl methylcellulose phthalate (HPMCP), methylcellulose succinate (MCS), or hydroxypropyl methylcellulose acetate succinate (HPMCAS). The esterified cellulose ethers are used for coating dosage forms, such as tablets, microparticulates or capsules. Enteric polymers protect the drug from inactivation or degradation in the acidic environment or prevent irritation of the stomach by the drug, but are dissolved in the intestinal canals to release the drug contained therein. US Patent No. 4,365,060 discloses enterosoluble capsules which are said to have excellent enterosolubility behavior.

US 7,138,143 discloses a coating dispersion soluble in the lower digestive tract which is prepared by blending a hydroxypropyl methylcellulose acetate succinate (HPMCAS) soluble at around pH 7 with a conventional plasticizer and an anion surfactant and further adding an acid, wherein the HPMCAS has an average particle size of 10 mum or less and is dispersed at a concentration of 2 to 20% by weight, and the acid is used in an amount of 1 to 10 parts by weight per 100 parts by weight of HPMCAS, and a sustained release coated preparation capable of releasing a medicament in the large intestine at the lower digestive tract, which is prepared by coating a medicament-containing solid preparation such as a granular core with the coating dispersion.

Enteric coatings or capsules can be prepared from organic or aqueous solutions of esterified cellulose ethers. European Patent Applications EP 0 662 323 and EP 0 677 322 disclose methods of preparing an aqueous emulsion for coating solid pharmaceutical preparations wherein a cellulosic polymer is dissolved in an organic solvent miscible with water or in a mixture of the organic solvent with water to give a polymer solution having a polymer concentration of not more than 10 wt. %, the solution is mixed with (additional) water to disperse the solution in water, and then organic solvent is removed. The published Japanese Patent Application JP8109124-A discloses the production of coating powders from such emulsions by adding an anionic surfactant and spray-drying. However, organic solvents are often not desirable for pharmaceutical or nutritional uses. On the other hand, esterified cellulose ethers only have a limited solubility in water.

The published Japanese Patent Application JP7070203A discloses a process wherein a hydroxycarboxylic acid type cellulose derivative is spread in water and pulverised by a pulveriser having a specific design to produce a cellulose derivative having a mean particle size below 7 microns, especially below 5 microns.

European Patent Application EP 0 648 487 discloses an aqueous dispersion comprising 5 to 15 wt. % of an enteric coating base, such as HPMCAS or HPMCP. The aqueous dispersion further comprises 15 - 40 wt.% of a plasticizer, such as triethyl citrate or triacetin, and 0.1 - 10 wt.% of an anionic surfactant, such as sodium alkyl sulfate, or a sodium or potassium salt of a fatty acid, such as sodium oleate or potassium sorbate, based on the weight of HPMCAS or HPMCP.

International Patent Application WO 2013/164122 discloses an aqueous composition for the manufacture of capsule shells comprising 5 - 50 wt. % of a wide range of functional polymers. In the majority of the examples the capsules are produced from an Aquacoat CPD 30 dispersion, which is a 30 wt. % aqueous dispersion comprising 23 wt.% non-salified cellulose acetate phthalate (CAP) and 7 wt. % Poloxamer, optionally blended with a minor amount of a HPMCAS slurry. Often uniform films can be obtained. A HPMCAS dispersion comprising 14% solids is also disclosed. Although 20% triethyl citrate is used as a film forming aid, when pins are heated to 50 °C and dipped into the dispersion, the HPMCAS polymer aggregates but the film rapidly collapses and flows down.

It would be desirable to provide an aqueous dispersion which comprises more than the 15 or 14 wt. % of HPMCAS or HPMCP as disclosed in EP-A- 0 648 487 and in WO 2013/164122. A high concentration of HPMCAS or HPMCP would be desirable to increase the efficiency of preparing films and capsules. An increased concentration of HPMCAS or HPMCP would reduce the amount of liquid diluent that needs to be removed, e.g. by evaporation, when preparing films and capsules. Unfortunately, an increased content of HPMCAS solids in the aqueous dispersion has the disadvantage of an increased viscosity at room temperature, i.e. at 20 °C. However, a sufficiently low viscosity at about 20 °C is highly desirable. The dispersion should have a reasonably good flowability at 20 °C to facilitate its handling.

Cooling the dispersion below 20 °C adds complexity and increases energy costs and is therefore not desirable.

HPMCAS particles generally become tacky and tend to agglomeration in aqueous dispersions at elevated temperatures. Finally the HPMCAS particles start to gel and the viscosity of the aqueous HPMCAS dispersion starts to significantly increase. The temperature at which the HPMCAS particles start to gel in the aqueous dispersion is designated hereafter as "phase transition temperature" of the dispersion.

Tackiness and agglomeration of HPMCAS particles and a low phase transition temperature of the dispersion, e.g., at 21 °C or less, is undesirable when the HPMCAS particles are subjected to high shear, e.g. when HPMCAS particles are to be mixed with other materials during preparation of HPMCAS dispersions or when the esterified cellulose ether particles are subjected to grinding in the presence of water to produce the aqueous dispersion. A phase transition temperature at 21 °C or less and excessively tacky esterified cellulose ether dispersions at high shear can lead to operational failures during production, such as plugging of the milling or mixing apparatus used for producing the esterified cellulose ether dispersions.

On the other hand, a viscosity increase of an aqueous dispersion comprising HPMCAS particles is often desirable when the aqueous dispersion is subjected to low shear, e.g. when the aqueous dispersion is gently agitated for producing coatings or for forming capsule shells on dipping pins. The viscosity increase of the aqueous dispersion improves adherence of the aqueous dispersion to a substrate, such as tablets to be coated or dipping pins.

Another important property of an aqueous composition comprising dispersed esterified cellulose ether particles is its storage stability. The size of the dispersed esterified cellulose ether particles preferably remain about the same over an extended time period, such as several weeks. When an aqueous dispersion of an esterified cellulose ether loses its storage stability, it solidifies accompanied by water exudation; then the dispersion loses its ability to flow under its own weight. It is preferred that an aqueous dispersion of an esterified cellulose ether can be stored for weeks or months while still maintaining its ability to flow under its own weight.

Fulfilling this wide variety of requirements is challenging. Much research effort has been spent on improving the known aqueous composition comprising dispersed esterified cellulose ethers.

One object of the present invention is to provide an aqueous composition comprising dispersed particles of an esterified cellulose ether which has a sufficiently low viscosity at a temperature of 20 °C to enable reasonably good flowability of the aqueous composition, even when the aqueous composition comprises at least 20 wt.% esterified cellulose ether. It is a preferred object of the present invention to provide an aqueous composition comprising dispersed esterified cellulose ethers particles which has a sufficiently low viscosity at a temperature of 20 °C, even when the aqueous composition comprises at least 25 wt.% or even at least 30 wt.% , or in preferred embodiments even at least 35 wt.% esterified cellulose ether particles.

It is another preferred object of the present invention to provide an aqueous dispersion comprising esterified cellulose ether particles, such as HPMCAS particles, which has a phase transition temperature above 20 °C.

It is another preferred object of the present invention to provide an aqueous dispersion comprising esterified cellulose ether particles which has good storage stability.

SUMMARY

One aspect of the present invention is an aqueous composition which comprises

> a) at least 25 percent, based on the total weight of the aqueous composition, of a dispersed esterified cellulose ether comprising (i) groups of the formula -C(O)-R-COOA or (ii) a combination of aliphatic monovalent acyl groups and groups of the formula -C(O)-R-COOA, wherein R is a divalent aliphatic or aromatic hydrocarbon group and A is hydrogen or a cation,
> b) from 0.1 to 15 percent of a salt of a fatty acid, based on the weight of the dispersed esterified cellulose ether, and
> c) from 0.02 to 7 percent of an anionic surfactant comprising a sulfate or sulfonate group, based on the weight of the dispersed esterified cellulose ether.

Another aspect of the present invention is a process for producing the above-mentioned aqueous composition, wherein the process comprises the steps of grinding, in the presence of an aqueous diluent, an esterified cellulose ether comprising (i) groups of the formula -C(O)-R-COOA or (ii) a combination of aliphatic monovalent acyl groups and groups of the formula -C(O)-R-COOA, wherein R is a divalent aliphatic or aromatic hydrocarbon group and A is hydrogen or a cation,

blending a salt of a fatty acid, an anionic surfactant comprising a sulfate or sulfonate group and optionally one or more adjuvants with the esterified cellulose ether before, during or after the grinding of the esterified cellulose ether, and choosing the amounts of aqueous diluent, esterified cellulose ether, salt of a fatty acid, anionic surfactant comprising a sulfate or sulfonate group and optionally one or more adjuvants such that the produced aqueous composition comprises a) at least 25 percent, based on the total weight of the aqueous composition, of the dispersed esterified cellulose ether, b) from 0.1 to 15 percent of the salt of a fatty acid, and c) from 0.02 to 7 percent of the anionic surfactant comprising a sulfate or sulfonate group, the percentages of components b) and c) being based on the weight of the dispersed esterified cellulose ether.

Yet another aspect of the present invention is a process for producing the above-mentioned aqueous composition, wherein the process comprises the steps of melting an esterified cellulose ether comprising (i) groups of the formula -C(O)-R-COOA or (ii) a combination of aliphatic monovalent acyl groups and groups of the formula -C(O)-R-COOA, wherein R is a divalent aliphatic or aromatic hydrocarbon group and A is hydrogen or a cation, and

emulsifying the molten esterified cellulose ether in an aqueous diluent, adding a salt of a fatty acid and an anionic surfactant comprising a sulfate or sulfonate group and optionally one or more adjuvants before, during or after the step of emulsifying the molten esterified cellulose ether in the aqueous diluent,

choosing the amounts of aqueous diluent, esterified cellulose ether, salt of a fatty acid, anionic surfactant comprising a sulfate or sulfonate group and optionally one or more adjuvants such that the produced aqueous composition comprises a) at least 25 percent, based on the total weight of the aqueous composition, of the dispersed esterified cellulose ether, b) from 0.1 to 15 percent of the salt of a fatty acid, and c) from 0.02 to 7 percent of the anionic surfactant comprising a sulfate or sulfonate group, the percentages of components b) and c) being based on the weight of the dispersed esterified cellulose ether,

and cooling the emulsion to form an aqueous dispersion.

Yet another aspect of the present invention is a dosage form which is coated with a coating prepared from the above-mentioned aqueous composition.

Yet another aspect of the present invention is a capsule shell which is made from the above-mentioned aqueous composition.

Yet another aspect of the present invention is a process for producing a capsule shell which comprises the steps of providing the above-mentioned aqueous composition, pre-heating molding pins to a temperature higher than the aqueous composition, dipping the pre-heating molding pins into the aqueous composition, forming a film on said molding pins by withdrawing said pins from said aqueous composition, and drying the film on the molding pins.

BRIEF DESCRIPTION OF DRAWINGS

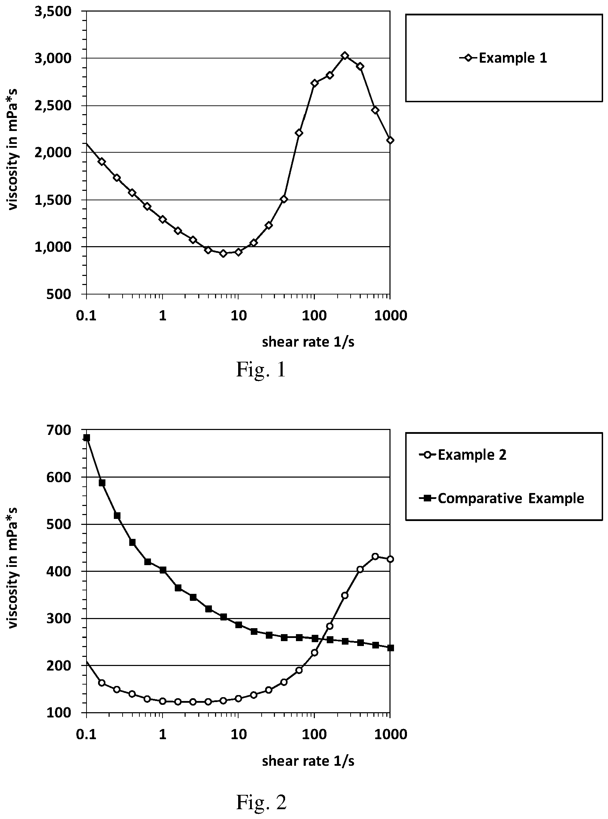

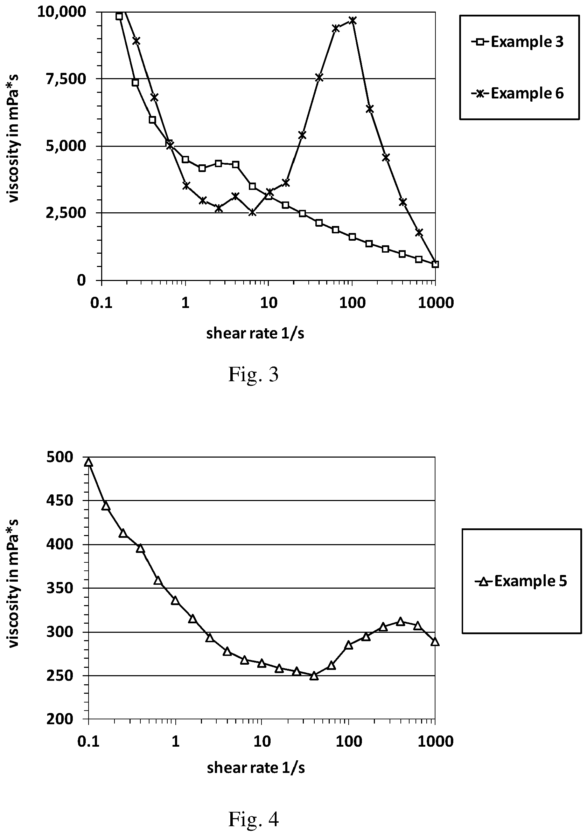

Figures 1 - 4 illustrate the viscosity of five aqueous compositions of the present invention and of a comparative aqueous composition depending on the shear rate at which the compositions are sheared.

DESCRIPTION OF EMBODIMENTS

Surprisingly, it has been found that new aqueous compositions can be provided which comprise at least 25 wt.%, preferably at least 30 wt.%, and in the most preferred embodiments even at least 35 wt.% of dispersed particles of an esterified cellulose ether as described below and which have a reasonably low viscosity at a temperature of 20 °C by incorporating into the aqueous composition from 0.1 to 15 percent of a salt of a fatty acid and from 0.02 to 7 percent of an anionic surfactant comprising a sulfate or sulfonate group, based on the weight of the dispersed esterified cellulose ether. A considerably higher concentration of particles of esterified cellulose ether can be incorporated into the aqueous composition while still maintaining a reasonable viscosity than when the composition comprises a comparable amount of another surfactant.

Surprisingly, it has also been found that the new aqueous compositions as described below do not exhibit undue tackiness or agglomeration of the esterified cellulose ether particles during preparation of the aqueous compositions under high shear, for example by grinding esterified cellulose ether particles in the presence of water, a salt of a fatty acid and an anionic surfactant comprising a sulfate or sulfonate group, at a temperature above 30 °C or even above 40 °C, and generally up to 50 °C.

The production of aqueous compositions comprising dispersed particles of an esterified cellulose ether is commonly done under high shear, which typically leads to the incorporation of air bubbles in the aqueous composition. It is highly desirable to minimize the content of air bubbles in the aqueous compositions, e.g. when they are used for producing capsules and coatings. It has surprisingly been found that less air bubbles are formed and/or formed air bubbles can be more easily removed from such compositions when the compositions comprise a surfactant combination of i) a salt of a fatty acid and ii) an anionic surfactant comprising a sulfate or sulfonate group than when the composition only comprises a salt of a fatty acid.

Moreover, the aqueous compositions of the present invention have a good storage stability.

Surprisingly, it has also been found that preferred embodiments of the aqueous compositions of the present invention as described below exhibit dilatancy at low shear, i.e., the viscosity of the aqueous composition increases at constant temperature when increasing shear rate within a certain range. "Low shear" as used herein means a shear rate of not more than about 1000 sec$^{-1}$. Low shear is commonly applied in processes for coating substrates, such as tablets, or when dipping pins into the aqueous composition for preparing capsules. The viscosity increase at constant temperature, e.g. at 21 °C, is of great advantage as it increases the adherence of the composition to the surface of the substrate to be coated, such as tablets, or to dipping pins in the case of capsule production.

The esterified cellulose ether comprised in the composition of the present invention has a cellulose backbone having β-1,4 glycosidically bound D-glucopyranose repeating units, designated as anhydroglucose units in the context of this invention. The esterified cellulose ether preferably is an esterified alkyl cellulose, hydroxyalkyl cellulose or hydroxyalkyl alkylcellulose. This means that in the esterified cellulose ether comprised in the composition of the present invention at least a part of the hydroxyl groups of the anhydroglucose units are substituted by alkoxyl groups or hydroxyalkoxyl groups or a combination of alkoxyl and hydroxyalkoxyl groups. The hydroxyalkoxyl groups are typically hydroxymethoxyl, hydroxyethoxyl and/or hydroxypropoxyl groups. Hydroxyethoxyl and/or hydroxypropoxyl groups are preferred. Typically one or two kinds of hydroxyalkoxyl groups are present in the esterified cellulose ether. Preferably a single kind of hydroxyalkoxyl group, more preferably hydroxypropoxyl, is present. The alkoxyl groups are typically methoxyl, ethoxyl and/or propoxyl groups. Methoxyl groups are preferred. Illustrative of the above-defined esterified cellulose ethers are esterified alkylcelluloses, such as esterified methylcelluloses, ethylcelluloses, and propylcelluloses; esterified hydroxyalkylcelluloses, such as esterified hydroxyethylcelluloses, hydroxypropylcelluloses, and hydroxybutylcelluloses; and esterified hydroxyalkyl alkylcelluloses, such as esterified hydroxyethyl methylcelluloses, hydroxymethyl ethylcelluloses, ethyl hydroxyethylcelluloses, hydroxypropyl methylcelluloses, hydroxypropyl ethylcelluloses, hydroxybutyl methylcelluloses, and hydroxybutyl ethylcelluloses; and those having two or more hydroxyalkyl groups, such as esterified hydroxyethylhydroxypropyl methylcelluloses. Most preferably, the esterified cellulose ether is an esterified hydroxyalkyl methylcellulose, such as an esterified hydroxypropyl methylcellulose.

The degree of the substitution of hydroxyl groups of the anhydroglucose units by hydroxyalkoxyl groups is expressed by the molar substitution of hydroxyalkoxyl groups, the MS(hydroxyalkoxyl). The MS(hydroxyalkoxyl) is the average number of moles of hydroxyalkoxyl groups per anhydroglucose unit in the esterified cellulose ether. It is to be understood that during the hydroxyalkylation reaction the hydroxyl group of a hydroxyalkoxyl group bound to the cellulose backbone can be further etherified by an alkylating agent, e.g. a methylating agent, and/or a hydroxyalkylating agent. Multiple subsequent hydroxyalkylation etherification reactions with respect to the same carbon atom position of an anhydroglucose unit yields a side chain, wherein multiple hydroxyalkoxyl groups are covalently bound to each other by ether bonds, each side chain as a whole forming a hydroxyalkoxyl substituent to the cellulose backbone.

The term "hydroxyalkoxyl groups" thus has to be interpreted in the context of the MS(hydroxyalkoxyl) as referring to the hydroxyalkoxyl groups as the constituting units of hydroxyalkoxyl substituents, which either comprise a single hydroxyalkoxyl group or a side chain as outlined above, wherein two or more hydroxyalkoxyl units are covalently bound to each other by ether bonding. Within this definition it is not important whether the terminal hydroxyl group of a hydroxyalkoxyl substituent is further alkylated or not; both alkylated and non-alkylated hydroxyalkoxyl substituents are included for the determination of MS(hydroxyalkoxyl). The esterified cellulose ether generally has a molar substitution of hydroxyalkoxyl groups of at least 0.05, preferably at least 0.08, more preferably at least 0.12, and most preferably at least 0.15. The degree of molar substitution is generally not more than 1.00, preferably not more than 0.90, more preferably not more than 0.70, and most preferably not more than 0.50.

The average number of hydroxyl groups substituted by alkoxyl groups, such as methoxyl groups, per anhydroglucose unit, is designated as the degree of substitution of alkoxyl groups, DS(alkoxyl). In the above-given definition of DS, the term "hydroxyl groups substituted by alkoxyl groups" is to be construed within the present invention to include not only alkylated hydroxyl groups directly bound to the carbon atoms of the cellulose backbone, but also alkylated hydroxyl groups of hydroxyalkoxyl substituents bound to the cellulose backbone. The esterified cellulose ethers preferably have a DS(alkoxyl) of at least 1.0, more preferably at least 1.1, even more preferably at least 1.2, most preferably at least 1.4, and particularly at least 1.6. The DS(alkoxyl) is preferably not more than 2.5, more preferably not more than 2.4, even more preferably not more than 2.2, and most preferably not more than 2.05.

Most preferably the esterified cellulose ether is an esterified hydroxypropyl methylcellulose having a DS(methoxyl) within the ranges indicated above for DS(alkoxyl) and an MS(hydroxypropoxyl) within the ranges indicated above for MS(hydroxyalkoxyl).

The esterified cellulose ether utilized in the present invention has (i) groups of the formula -C(O)-R-COOA or (ii) a combination of aliphatic monovalent acyl groups and groups of the formula -C(O)-R-COOA, wherein R is a divalent aliphatic or aromatic hydrocarbon group and A is hydrogen or a cation. The cation preferably is an ammonium cation, such as $NH_4^+$ or an alkali metal ion, such as the sodium or potassium ion, more preferably the sodium ion. Most preferably, A is hydrogen.

The aliphatic monovalent acyl groups are preferably selected from the group consisting of acetyl, propionyl, and butyryl, such as n-butyryl or i-butyryl.

Preferred groups of the formula -C(O)-R-COOA are -C(O)-CH$_2$-CH$_2$-COOA, such as -C(O)-CH$_2$-CH$_2$-COOH or - C(O)-CH$_2$-CH$_2$-COO$^-$Na$^+$, -C(O)-CH=CH-COOA, such as -C(O)-CH=CH-COOH or -C(O)-CH=CH-COO$^-$Na$^+$, or -C(O)-C$_6$H$_4$-COOA, such as -C(O)-C$_6$H$_4$-COOH or -C(O)-C$_6$H$_4$-COO$^-$Na$^+$.

In the groups of formula -C(O)-C$_6$H$_4$-COOA the carbonyl group and the carboxylic group are preferably arranged in ortho-positions.

Preferred esterified cellulose ethers are

i) HPMCXY, wherein HPMC is hydroxypropyl methyl cellulose, X is A (acetate), or X is B (butyrate) or X is Pr (propionate) and Y is S (succinate), or Y is P (phthalate) or Y is M (maleate), such as hydroxypropyl methyl cellulose acetate phthalate (HPMCAP), hydroxypropyl methyl cellulose acetate maleate (HPMCAM), or hydroxypropyl methylcellulose acetate succinate (HPMCAS), or

ii) hydroxypropyl methyl cellulose phthalate (HPMCP), hydroxypropyl cellulose acetate succinate (HPCAS), hydroxybutyl methyl cellulose propionate succinate (HBMCPrS), hydroxyethyl hydroxypropyl cellulose propionate succinate (HEHPCPrS); and methyl cellulose acetate succinate (MCAS).

Hydroxypropyl methylcellulose acetate succinate (HPMCAS) is the most preferred esterified cellulose ether.

The esterified cellulose ethers generally have a degree of substitution of aliphatic monovalent acyl groups, such as acetyl, propionyl, or butyryl groups, of not more than 1.75, preferably not more than 1.50, more preferably not more than 1.25, and most preferably not more than 1.00, or even not more than 0.65. The degree of substitution of aliphatic monovalent acyl groups can be zero, but preferably it is at least 0.05, more preferably at least 0.10, and most preferably at least 0.20.

The esterified cellulose ethers generally have a degree of substitution of groups of formula -C(O)-R-COOA, such as succinoyl, of at least 0.05, preferably at least 0.10. The degree of substitution of groups of formula -C(O)-R-COOA generally is up to 1.6, preferably up to 1.30, more preferably up to 1.00, and most preferably up to 0.70 or even up to 0.60.

The sum of i) the degree of substitution of aliphatic monovalent acyl groups and ii) the degree of substitution of groups of formula -C(O)-R-COOA is generally at least 0.05, preferably at least 0.10, more preferably at least 0.20, most preferably at least 0.30, and particularly at least 0.40. The mentioned sum is generally no more than 2.0, preferably no more than 1.4, more preferably no more than 1.15, most preferably no more than 1.10 and particularly no more than 1.00.

The content of the acetate and succinate ester groups is determined according to "Hypromellose Acetate Succinate", United States Pharmacopeia and National Formulary, NF 29, pp. 1548-1550. Reported values are corrected for volatiles (determined as described in section "loss on drying" in the above HPMCAS monograph). The method may be used in analogue manner to determine the content of propionyl, butyryl, phthalyl and other ester groups.

The content of ether groups in the esterified cellulose ether is determined in the same manner as described for "Hypromellose", United States Pharmacopeia and National Formulary, USP 35, pp 3467-3469.

The contents of ether and ester groups obtained by the above analyses are converted to DS and MS values of individual substituents according to the formulas below. The formulas may be used in analogue manner to determine the DS and MS of substituents of other cellulose ether esters.

$$
\begin{aligned}
\% \text{ cellulose backbone} \\
= 100 - &\left( \%\text{MeO} * \frac{M(OCH_3) - M(OH)}{M(OCH_3)} \right) \\
- &\left( \%\text{HPO} * \frac{M(OCH_2CH(OH)CH_3) - M(OH)}{M(OCH_2CH(OH)CH_3)} \right) \\
- &\left( \%\text{Acetyl} * \frac{M(COCH_3) - M(H)}{M(COCH_3)} \right) \\
- &\left( \%\text{Succinoyl} * \frac{M(COC_2H_4COOH) - M(H)}{M(COC_2H_4COOH)} \right)
\end{aligned}
$$

$$
DS(Me) = \frac{\dfrac{\%\text{MeO}}{M(OCH_3)}}{\dfrac{\%\text{cellulose backbone}}{M(AGU)}}
\qquad\qquad
MS(HP) = \frac{\dfrac{\%\text{HPO}}{M(HPO)}}{\dfrac{\%\text{cellulose backbone}}{M(AGU)}}
$$

$$DS(Acetyl) = \frac{\frac{\%Acetyl}{M(Acetyl)}}{\frac{\%cellulose\ backbone}{M(AGU)}} \qquad DS(Succinoyl) = \frac{\frac{\%Succinoyl}{M(Succinoyl)}}{\frac{\%cellulose\ backbone}{M(AGU)}}$$

$M(MeO) = M(OCH_3) = 31.03\ Da\ M(HPO) = M(OCH_2CH(OH)CH_3) = 75.09\ Da\ M(Acetyl) = M(COCH_3)\ M(Succinoyl) = M(COC_2H_4COOH) = 43.04\ Da = 101.08\ Da\ M(AGU) = 162.14\ Da\ M(OH) = 17.008\ Da\ M(H) = 1.008\ Da$

By convention, the weight percent is an average weight percentage based on the total weight of the cellulose repeat unit, including all substituents. The content of the methoxyl group is reported based on the mass of the methoxyl group (i.e., -OCH$_3$). The content of the hydroxyalkoxyl group is reported based on the mass of the hydroxyalkoxyl group (i.e., -O-alkylene-OH); such as hydroxypropoxyl (i.e., +O-CH$_2$CH(CH$_3$)-OH). The content of the aliphatic monovalent acyl group is reported based on the mass of -C(O)-R$_1$ wherein R$_1$ is a monovalent aliphatic group, such as acetyl (-C(O)-CH$_3$). The content of the group of formula -C(O)-R-COOH is reported based on the mass of this group, such as the mass of succinoyl groups (i.e., -C(O)-CH$_2$-CH$_2$-COOH).

The esterified cellulose ether comprised in the composition of the present invention generally has a viscosity of at least 1.2 mPa·s, preferably least 1.8 mPa·s, and more preferably least 2.4 mPa·s, and generally no more than 200 mPa·s, preferably no more than 100 mPa·s, more preferably no more than 50 mPa·s, and most preferably no more than 30 mPa·s, measured as a 2.0 weight percent solution of the esterified cellulose ether in 0.43 wt% aqueous NaOH at 20°C according to "Hypromellose Acetate Succinate, United States Pharmacopia and National Formulary, NF 29, pp. 1548-1550".

The aqueous composition of the present invention comprises at least 25 percent, preferably at least 30 percent, and most preferably even at least 35 percent of the esterified cellulose ether(s) in dispersed state in the aqueous composition. The aqueous composition of the present invention generally comprises up to 40 percent or in some cases even up to 43 percent of the esterified cellulose ether(s) in dispersed state in the aqueous composition. These percentages are based on the total weight of the composition.

The aqueous composition further comprises from 0.1 to 15 percent of a salt of a fatty acid, and from 0.02 to 7 percent of an anionic surfactant comprising a sulfate or sulfonate group, each based on the weight of the dispersed esterified cellulose ether. The aqueous composition may comprise more than one salt of a fatty acid and/or more than one anionic surfactant comprising a sulfate or sulfonate group, provided that the total amount of fatty acid salts and the total amount of anionic surfactants comprising a sulfate or sulfonate group are within the above-mentioned weight ranges.

The total amount of the salt(s) of a fatty acid is at least 0.1 percent, preferably at least 0.5 percent, most preferably at least 1.0 percent, and particularly at least 2.0 percent, based on the total weight of the esterified cellulose ether(s). The total amount of the salt(s) of a fatty acid is up to 15 percent, preferably up to 10 percent, most preferably up to 7.0 percent, and particularly only up to 5.0 percent, based on the total weight of the esterified cellulose ether(s).

Preferred fatty acid salts are ammonium, alkali metal or alkaline earth metal salts. A preferred ammonium ion is NH$_4^+$. Preferred alkali metal ions are the sodium or potassium ions. A preferred alkaline earth metal ion is the calcium ion. The fatty acids can be saturated or unsaturated. Exemplary of saturated fatty acids are caprylic acid, capric acid, lauric acid, myristic acid, palmitic acid, stearic acid, arachidic acid, behenic acid, lignoceric acid and cerotic acid. The unsaturated fatty acids can be mono-, di- or triunsaturated fatty acids, mono-unsaturated and di- unsaturated fatty acids being preferred. Exemplary of mono-unsaturated fatty acids are myristoleic acid, palmitoleic acid, sapienic acid, oleic acid, elaidic acid and vaccenic acid. Exemplary of di-unsaturated fatty acids are linoleic acid and linoelaidic acid. Ammonium, alkali metal and alkaline earth metal salts of stearic acid or oleic acid are most preferred, particularly those salts mentioned above.

The total amount of anionic surfactant(s) comprising a sulfate or sulfonate group is at least 0.02 percent, preferably at least 0.05 percent, most preferably at least 0.1 percent, and particularly at least 0.2 percent, based on the total weight of the esterified cellulose ether(s). The total amount of the anionic surfactant(s) comprising a sulfate or sulfonate group is up to 7 percent, preferably up to 5 percent, most preferably up to 3 percent, and particularly only up to 1.5 percent, based on the total weight of the esterified cellulose ether(s).

Preferred are ammonium, alkali metal or alkaline earth metal salts of the anionic surfactant comprising a sulfate or sulfonate group. The anionic surfactant can have one or more, preferably one or two, sulfate or sulfonate groups. A preferred ammonium ion is NH$_4^+$. Preferred alkali metal ions are the sodium or potassium ions. A preferred alkaline earth metal ion is the calcium ion. Preferably the anionic surfactant comprises one or more, preferably one or two, alkyl, alkenyl, alkinyl or cycloalkyl groups. Preferably the alkyl, alkenyl, alkinyl or cycloalkyl groups each independently comprise from 4 to 12 carbon atoms, more preferably from 6 to 8 carbon atoms. The total number of carbon atoms in the alkyl, alkenyl, alkinyl or cycloalkyl group(s) in the surfactant is preferably 8 to 24, more preferably 12 to 16. These groups can be branched or linear. Preferred anionic surfactants comprising a sulfate or sulfonate group are alkyl sulfate salts, such as ammonium lauryl sulfate, potassium lauryl sulfate, sodium dodecyl sulfate (SDS) or sodium laureth sulfate (SLES);

or alkyl benzene sulphonates, such as sodium dodecylbenzenesulfonate, or alkyldiphenyloxide disulfonate salts, such as sodium dodecyl diphenyl ether disulfonate. More preferred anionic surfactants comprising a sulfate or sulfonate group are dialkyl, dialkenyl, dialkinyl or dicycloalkyl sulfosuccinates. Most preferred is a dialkyl sulfosuccinate, particularly dioctyl sodium sulfosuccinate (IUPAC name sodium 1,4-bis(2-ethylhexoxy)-1,4-dioxobutane-2-sulfonate), dioctyl potassium sulfosuccinate, or dioctyl calcium sulfosuccinate.

The aqueous composition of the present invention is in the form of an aqueous dispersion, typically in the form of a stable dispersion. The median particle size, d50, of the dispersed esterified cellulose ether particles is typically up to 7 micrometers, more typically up to 5 micrometers, and even more typically up to 4 micrometers. The median particle size, d50, of the dispersed esterified cellulose ether particles is typically 0.3 micrometers or more, more typically 1.0 micrometers or more, and most typically 1.5 micrometers or more. The particle size is measured by laser diffraction particle size analysis, e.g., using a Beckman Coulter laser diffraction particle size analyzer which is commercially available from Beckman Coulter, California. The median particle size d50 is the diameter where 50 volume percent of the particles have a smaller equivalent diameter and 50 volume percent have a larger equivalent diameter. Typically d90 is 1.0 micrometers or more, more typically 2.0 micrometers or more, and most typically 4.0 micrometers or more; and typically up to 12 micrometers, more typically up to 10 micrometers, and most typically up to 7 micrometers, d90 being the diameter where 90 volume percent of the particles have a smaller equivalent diameter and the other 10 volume percent have a larger equivalent diameter. The equivalent particle diameter d is the diameter of a sphere having the same volume as the volume of a given particle. The mean particle diameter is typically 0.5 micrometers or more, more typically 1.0 micrometers or more, and most typically 2.0 micrometers or more; and typically up to 8 micrometers, more typically up to 6 micrometers, and most typically even only up to 4 micrometers.

The aqueous composition of the present invention comprises an aqueous diluent. The aqueous diluent is water, optionally mixed with a minor amount of an organic solvent. The aqueous diluent preferably consists of 50 - 100 weight percent, more preferably 65 - 100 weight percent, and most preferably 75 - 100 weight percent of water and preferably 0 - 50 weight percent, more preferably 0 - 35 weight percent, and most preferably 0 - 25 weight percent of an organic solvent, based on the total weight of water and the organic solvent. Useful organic solvents are polar organic solvents having one or more heteroatoms, such as oxygen, nitrogen or halogen like chlorine. More preferred organic solvents are alcohols, for example multifunctional alcohols, such as glycerol, or preferably monofunctional alcohols, such as methanol, ethanol, isopropanol or n-propanol; ethers, such as tetrahydrofuran, ketones, such as acetone; methyl ethyl ketone, or methyl isobutyl ketone; acetates, such as ethyl acetate; halogenated hydrocarbons, such as methylene chloride; or nitriles, such as acetonitrile. Preferably the aqueous composition of the present invention comprises water alone as aqueous diluent. The amount of the aqueous diluent is typically at least 50 percent, more typically at least 55 percent, based on the total weight of the aqueous composition. The amount of the aqueous diluent is typically no more than 79 percent, more typically no more than 75 percent, based on the total weight of the aqueous composition.

The apparent viscosity of the aqueous composition of the present invention is generally 50 mPa·s or more, typically 100 mPa·s or more, measured at 20 ° C. The apparent viscosity of the aqueous composition of the present invention is generally no more than 5000 mPa·s, typically no more than 4000 mPa·s, measured at 20 ° C.

The aqueous composition of the present invention may further comprise optional ingredients, for example active ingredients, such as fertilizers, herbicides, pesticides, or biologically active ingredients, such as vitamins, herbals or mineral supplements or drugs; or adjuvants such as one or more plasticizers, film forming aids, coloring agents, pigments, opacifiers, flavor or taste improvers, antioxidants, or any combination thereof. Optional additives are preferably pharmaceutically acceptable. The amount of these optional ingredients is typically from 0 to 50 percent of the total weight of the ingredients of the aqueous composition excluding the aqueous diluent. Typically the amount is 1 percent or more, more typically 5 percent or more; and typically up to 40 percent, more typically up to 20 percent of the total weight of the ingredients of the aqueous composition excluding the aqueous diluent.

In one embodiment, the aqueous composition of the present invention further comprises at least one film forming aid. The term "film forming aid" comprises one or more plasticizers conventionally used in the manufacture of coatings or capsule shells, notably hard capsule shells, to ensure the formation of self-supported cohesive films and avoid capsule brittleness, and/or one or more viscosity enhancers at elevated temperature, i.e. natural as well as synthetic substances conventionally used to optimize aqueous compositions for coating purposes or the dip molding manufacture of hard capsule shells.

Film forming aids that display plasticizing properties include: phthalic esters, such as dimethyl-, diethyl-, and diisopropyl-phthalate; citric esters, such as triethyl-, tributyl-, acetyltriethyl- and acetyltributyl-citrate; phosphoric esters, such as triethyl-, tricresyl, and triphenyl-phosphate; alkyl lactate; glycol esters; glycerol and glycerol esters, such as glycerol triacetate also known as triacetine; sucrose esters; oils and fatty acid esters; butyl stearate; dibutyl sebacate; dibutyl tartrate; diisobutyl adipate, tributyrin; propylene glycol; and mixtures thereof.

In one embodiment, film forming aids are cellulose ethers, such as carboxy methylcellulose, hydroxypropyl cellulose, ethyl cellulose, methylcellulose, hydroxypropylmethylcellulose (HPMC), e.g. HPMC types 2910, 2906 and/or 2208 as defined in USP30- NF25; gelatin, pullulan, non enteric starch derivatives, such as hydroxypropyl starch; polyvinyl acetate derivatives (PVAP); sorbitan monoesters; sorbitan polyoxyethylene esters; fatty acid esters; glycerol polyethylene, glycol ricinoleate; macrogolglycerides; triethyl citrate (TEC); acetyl trialkyl citrate; glycerol triacetate (triacetine); talc; and mixtures thereof.

In one embodiment the aqueous composition of the present invention comprises at least 5 percent, more preferably at least 10 percent, even more preferably at least 13 percent, and most preferably at least 15 percent of one or more film forming aids, such as plasticizers, based on the weight of the dispersed esterified cellulose ether. The amount of said one or more film forming aids, such as plasticizers, is generally up to 30 percent, preferably up to 25 percent, even more preferably up to 22 percent, and most preferably up to 20 percent, based on the weight of the dispersed esterified cellulose ether.

The aqueous composition of the present invention can be prepared by various methods. One method includes grinding the esterified cellulose ether in the presence of an aqueous diluent and optionally in the presence of one or more adjuvants. Another method includes melting or softening the esterified cellulose ether at an elevated temperature, optionally in the presence of one or more adjuvants, and emulsifying the molten or softened mass in the aqueous diluent. Suitable and preferred types and amounts of esterified cellulose ethers, salts of fatty acids, anionic surfactants comprising a sulfate or sulfonate group, optional adjuvants and aqueous diluents in the processes for producing the aqueous composition of the present invention are described further above.

Preparing an aqueous composition by simply physically blending an esterified cellulose ether, a salt of a fatty acid, an anionic surfactant comprising a sulfate or sulfonate group and an aqueous diluent at room temperature is not suitable for preparing a stable dispersion.

In one embodiment the process for producing the aqueous composition of the present invention comprises the steps of grinding, in the presence of an above-described aqueous diluent, at least one esterified cellulose ether as described above, blending a salt of a fatty acid, an anionic surfactant comprising a sulfate or sulfonate group and optionally one or more adjuvants with the esterified cellulose ether before, during or after the grinding of the esterified cellulose ether, and choosing the amounts of aqueous diluent, esterified cellulose ether, salt of a fatty acid, anionic surfactant comprising a sulfate or sulfonate group and optionally one or more adjuvants such that the produced aqueous composition comprises a) at least 25 percent, based on the total weight of the aqueous composition, of the dispersed esterified cellulose ether, b) from 0.1 to 15 percent of the salt of a fatty acid, and c) from 0.02 to 7 percent of the anionic surfactant comprising a sulfate or sulfonate group, the percentages of components b) and c) being based on the weight of the dispersed esterified cellulose ether. The salt of a fatty acid, the anionic surfactant comprising a sulfate or sulfonate group and optional adjuvants are preferably added before or during the grinding of the esterified cellulose ether in the aqueous diluent. A salt of a fatty acid, an anionic surfactant comprising a sulfate or sulfonate group and optional adjuvants can also be added after the grinding of the esterified cellulose ether, but generally at least 50 percent of the salt of a fatty acid that is used for preparing the aqueous composition of the present invention is added before or during the grinding of the esterified cellulose ether.

Any grinding device suitable for grinding esterified cellulose ethers in the presence of an aqueous diluent to a median particle size d50 as indicated further above can be used. Preferred grinding devices are wet grinding units such as media mills or bead mills. The grinding is typically conducted at a temperature of at least 10 °C, preferably of at least 30 °C, and typically at a temperature of up to 55 °C, more typically up to 50 °C. Grinding is generally conducted for a sufficient time period to achieve an above-mentioned median particle size, d50, of the dispersed esterified cellulose ether particles. It has surprisingly been found that improved storage stability of the aqueous composition of the present invention is achieved when the esterified cellulose ether, the salt of a fatty acid and optionally the anionic surfactant comprising a sulfate or sulfonate group are ground together in the presence of an aqueous diluent at a temperature of from 37 to 55 °C, more preferably at a temperature of from 40 to 55 °C. The grinding period is typically from 60 to 180 minutes. An aqueous dispersion of an esterified cellulose ether that has been prepared in such a manner can usually be stored more than 3 months, in the most preferred embodiments even more than 6 months, without losing its ability to flow under its own weight if it do not comprise a film forming aid.

In another embodiment the process for producing the aqueous composition of the present invention comprises the steps of melting at least one esterified cellulose ether as described above and emulsifying the molten esterified cellulose ether in an aqueous diluent, adding a salt of a fatty acid and an anionic surfactant comprising a sulfate or sulfonate group and optionally one or more adjuvants before, during or after the step of emulsifying the molten esterified cellulose ether in the aqueous diluent, choosing the amounts of aqueous diluent, esterified cellulose ether, salt of a fatty acid, anionic surfactant comprising a sulfate or sulfonate group and optionally one or more adjuvants such that the produced aqueous composition comprises a) at least 25 percent, based on the total weight of the aqueous composition, of the dispersed esterified cellulose ether, b) from 0.1 to 15 percent of the salt of a fatty acid, and c) from 0.02 to 7 percent of the anionic surfactant comprising a sulfate or sulfonate group, the percentages of components b) and c) being based on the weight of the dispersed esterified cellulose ether, and cooling the emulsion to form an aqueous dispersion. This embodiment of the process is preferably conducted in an extruder. Alternatively, a pressurized batch kneader can be used for conducting this embodiment of the invention. The general process conditions and equipment which are useful to perform the process are disclosed in U.S. Patent Nos. 5,539,021 and 7,763,676, the disclosure of which is incorporated herein by reference.

In another aspect of the invention the aqueous composition of the present invention may be used for coating dosage forms, such as tablets, granules, pellets, caplets, lozenges, suppositories, pessaries or implantable dosage forms, to form a coated composition. If the aqueous composition of the present invention comprises an active ingredient, such as a drug, drug layering can be achieved, i.e., the dosage form and the coating may comprise different active ingredients for different end-uses and/or having different release kinetics. The coating can be carried out in a known manner, for example by known dipping or spraying processes.

In yet another aspect of the invention the aqueous composition of the present invention may be used for the manufacture of capsules shells in a process which comprises the step of contacting the aqueous composition with dipping pins. According to one embodiment the process for producing capsule shells comprises the steps of providing the aqueous composition of the present invention as described above, pre-heating molding pins to a temperature higher than the aqueous composition, dipping the pre-heated molding pins into the aqueous composition, forming a film on said molding pins by withdrawing said pins from said aqueous composition, and drying the film on the molding pins. The general process conditions and equipment which may be used to prepare capsules shells are described in International Patent Application Nos. WO 2013/164122 and WO 2013/164121, the disclosures of which are incorporated herein by reference.

The aqueous composition of the present invention is particularly useful for coating dosage forms including tablets, capsules and others, or for the formation of capsules shells, all preferably for enteric use, i.e., coatings or capsules shells that are dissolved in the intestinal canals to release the active ingredient like a drug contained in the dosage form or in the capsules.

EXAMPLES

Some embodiments of the invention will now be described in detail in the Examples.

Unless otherwise mentioned, all parts and percentages are by weight. In the Examples the following test procedures are used.

Viscosity of Hydroxypropyl Methyl Cellulose Acetate Succinate (HPMCAS)

A 2.0 % by weight solution of the HPMCAS in 0.43 wt% aqueous NaOH was prepared as described in"Hypromellose Acetate Succinate, United States Pharmacopia and National Formulary, NF 29, pp. 1548-1550, followed by an Ubbelohde viscosity measurement at 20 °C according to DIN 51562-1:1999-01 (January 1999).

Content of ether and ester groups of HPMCAS

The content of ether groups in HPMCAS was determined in the same manner as described for "Hypromellose", United States Pharmacopeia and National Formulary, USP 35, pp 3467-3469. The ester substitution with acetyl groups ($-CO-CH_3$) and the ester substitution with succinoyl groups ($-CO-CH_2-CH_2-COOH$) were determined according to Hypromellose Acetate Succinate, United States Pharmacopia and National Formulary, NF 29, pp. 1548-1550". Reported values for ester substitution were corrected for volatiles (determined as described in section "loss on drying" in the above HPMCAS monograph).

Apparent viscosity of the aqueous composition comprising HPMCAS

The apparent viscosity of the aqueous dispersion comprising HPMCAS was measured at various temperatures according to a temperature sweep experiment performed with a Anton Paar MCR 301 rheometer with a CC-27 cup geometry and a 4-blade vane geometry ST26-4V-20 over a temperature range of 10 to 50 °C with a heating rate of 3 °C / min and a constant speed of the vane geometry of 40 rpm and a measurement point duration of 0.2721 min. Prior to this temperature sweep testing the material was treated with a SpeedMixer™ DAC 150.1 FV (FlackTek Inc.) at 2300 rpm for 1 min. A sample volume of 20 ml was used for these measurements. The samples had been stored at room temperature prior to the viscosity measurement.

Determination of phase transition temperature of the aqueous composition

The obtained data of the apparent viscosity, which was obtained from the temperature sweep measurements above, was used for this analysis. The phase transition temperature of the aqueous composition is the temperature at which the HPMCAS particles start to gel and the viscosity of the aqueous composition starts to significantly increase.

The average viscosity of the aqueous composition at temperatures from 10 to 15 °C was calculated from the measured apparent viscosity data to determine the baseline viscosity of the aqueous composition. The standard deviation of the baseline viscosity was calculated. When the standard deviation was more than 25 %, this was an indication that there was no constant viscosity at a temperature range from 10 to 15 °C and that the phase transition temperature was below 15 °C. The phase transition temperature of the aqueous composition was determined to be the temperature at which the viscosity of the aqueous composition reached 150% of its baseline viscosity.

Determination of viscosity of the aqueous dispersion depending on shear rate

The viscosity of the aqueous HPMCAS dispersion comprising HPMCAS was measured at 21 °C with a Haake RS600 rheometer (from Thermo Fischer Scientific, Karlruhe) with a cone & plate geometry (C-60/1°) in a steady shear flow curve experiment over a steady shear rate range from 0.1 - 1000 $s^{-1}$ with 5 data points each decade.

HPMCAS particle size measurement in the aqueous dispersion

To measure particle sizes 1-2 g of the aqueous HPMCAS dispersion that had been produced as described below was diluted in 20 ml of purified water. The particle size in the diluted dispersion was measured by laser diffraction particle size analysis using a Beckman Coulter LS 13 320 laser diffraction particle size analyzer which is commercially available from Beckman Coulter, California. The Universal Liquid Module (ULM) with a Fraunhofer optical model, a Polarization Intensity Differential Scattering (PIDS) system and a sonication control unit were used. In the sonication control unit the HPMCAS dispersion was subjected to ultrasonic treatment for a time period of up to 120 seconds during the HPMCAS addition (about 30 seconds) and particle size measurement (about 90 seconds).

Stability assessment

To assess the stability of the aqueous HPMCAS dispersion, the HPMCAS particle size measurement described above was repeated after about 2 weeks. The degree of changes in particle size was a clear indication of the stability of the aqueous HPMCAS dispersion. The dispersion was also visually inspected.

Determination of solids content

The solids content was determined using a moisture balance (Mettler Toledo Advanced Moisture Analyzer, Model HB43-S). Instrument settings were as follows: 3 g dispersion using the Rapid drying program with a temperature set point of 120 °C (40% overshoot for first 3 minutes) with switch-off criteria 5 (less than 1 mg weight change over 140 seconds). Upon drying to remove water, the residual solids content (including all additives) was weighed.

HPMCAS used for preparing the aqueous dispersion in Comp. Examples A - E and H

HPMCAS was used that had 23.7% methoxyl groups ($DS_{methoxyl}$ = 1.93), 7.1% hydroxypropoxyl groups ($MS_{hydroxypropoxyl}$ = 0.24), 9.6 % acetyl groups ($DS_{acetyl}$ = 0.56), 10.5% succinoyl groups ($DS_{succinoyl}$ = 0.26), and a viscosity of 2.96 mPa·s, measured as a 2.0% by weight solution of the HPMCAS in 0.43 wt.% aqueous NaOH.

HPMCAS used for preparing the aqueous dispersion in Comparative Example F and G

HPMCAS was used that had 23.3% methoxyl groups ($DS_{methoxyl}$ = 1.92), 7.2% hydroxypropoxyl groups ($MS_{hydroxypropoxyl}$ = 0.24), 9.8% acetyl groups ($DS_{acetyl}$ = 0.58), 10.9% succinoyl groups ($DS_{succinoyl}$ = 0.28), and a viscosity of 2.68 mPa·s, measured as a 2.0% by weight solution of the HPMCAS in 0.43 wt.% aqueous NaOH.

HPMCAS used in Comparative Example I, in Examples 1 and 2 and in Examples 8-10

HPMCAS was used that had 23.0 % methoxyl groups ($DS_{methoxyl}$ = 1.89), 7.3 % hydroxypropoxyl groups ($MS_{hydroxypropoxyl}$ = 0.25), 9.1 % acetyl groups ($DS_{acetyl}$ = 0.54), 11.6% succinoyl groups ($DS_{succinoyl}$ = 0.29), and a viscosity of 2.9 mPa·s, measured as a 2.0 % by weight solution of the HPMCAS in 0.43 wt.% aqueous NaOH.

HPMCAS used for preparing the aqueous dispersions in Examples 3 and 5

HPMCAS was used that had 23.2 % methoxyl groups ($DS_{methoxyl}$ = 1,90), 7.4 % hydroxypropoxyl groups ($MS_{hydroxypropoxyl}$ = 0.25), 9.4 % acetyl groups ($DS_{acetyl}$ = 0.56), 11.0% succinoyl groups ($DS_{succinoyl}$ = 0.28), and a viscosity of 3.0 mPa·s, measured as a 2.0 % by weight solution of the HPMCAS in 0.43 wt.% aqueous NaOH.

HPMCAS used for preparing the aqueous dispersions in Examples 4 and 7

HPMCAS was used that had 23.2% methoxyl groups ($DS_{methoxyl}$ = 1.91), 7.4% hydroxypropoxyl groups ($MS_{hydroxypropoxyl}$ = 0.25), 9.6 % acetyl groups ($DS_{acetyl}$ = 0.57), 11.0 % succinoyl groups ($DS_{succinoyl}$ = 0.28), and a viscosity of 2.9 mPa·s, measured as a 2.0 % by weight solution of the HPMCAS in 0.43 wt.% aqueous NaOH.

HPMCAS used for preparing the aqueous dispersions in Example 6

A mixture of the same HPMCAS as used in Examples 3 and 5 and another HPMCAS that had 23.0 % methoxyl groups ($DS_{methoxyl}$ = 1.89), 7.3 % hydroxypropoxyl groups ($MS_{hydroxypropoxyl}$ = 0.25), 9.8 % acetyl groups ($DS_{acetyl}$ = 0.58), 10.8 % succinoyl groups ($DS_{succinoyl}$ = 0.27), and a viscosity of 2.9 mPa·s, measured as a 2.0 % by weight solution of the HPMCAS in 0.43 wt.% aqueous NaOH was used.

Comparative Examples A - F

To produce an aqueous HPMCAS dispersion, water was loaded first and recirculated through a Netzsch LAB STAR media mill (1.4 mm Ytterum Stabilized Zirconia media, 0.7 mm screen size). During the milling process HPMCAS solids and a surfactant as listed in Table 1 below were loaded gradually to water recirculating through the mill at a mill speed of 3600 rev/min. The HPMCAS and the surfactant were added at a predetermined weight ratio to provide a percentage of surfactant, based on HPMCAS, as listed in Table 1 below. Addition of HPMCAS and surfactant was continued until a total solids loading of 20-30% was achieved, based on the total weight of the composition. The percentage of HPMCAS, based on the total weight of the composition, was calculated from the measured solids content and the given weight ratio between HPMCAS and the surfactant. The results are listed in Table 1 below. Following the addition of all solids, milling continued until the final particle size was obtained.

Comparative Examples A, B, D, E and F were completed using a similar procedure. In Comparative Example C no dispersion was obtained as the viscosity of the dispersion caused a pressure in the mill that exceeded the allowable pressure system.

In Comparative Example A no additive, i.e. no surfactant, was used. In Comparative Example B the well-known non-ionic surfactant polyoxyethylene (20) sorbitan monooleate, commercially available under the Trademark Tween 80 was used. In Comparative Examples C and D sodium dodecyl sulfate (SDS) was used, which is a well-known anionic surfactant. In Comparative Example E a block copolymer of ethylene oxide and propylene oxide, which is commercially available from BASF under the Trademark Pluronic L44 NF INH, was used as non-ionic surfactant.

The dispersion of Comparative Example B had HPMCAS particles that were very large. In Comparative Example C no dispersion was obtained as the viscosity of the dispersion caused a pressure in the mill that exceeded the allowable system pressure. The dispersions of Comparative Examples A, B and D produced a dispersion with acceptable viscosity and particle size but with tendency to agglomerate upon storage for about 2 weeks. The dispersions of Comparative Examples A - E were not suitable for reasonably convenient handling and processing.

The dispersion of Comparative Example F corresponds to Example 4 of the International co-pending patent application PCT/US15/018390 having a filing date of 03 March 2015 and a first priority date of 08 April 2014.

Comparative Examples G and H

Comparative Example F was repeated, except that a Drais DCP-12 Advantis media mill (1.0 mmYtterum Stabilized Zirconia media, 0.5 mm screen size) was used. The mill speed was initially set at 1600 rpm and then reduced as necessary down to about 1300 rpm to control the mill outlet temperature. The amount of sodium stearate and the final solids content were altered as shown in Table 1 below.

The dispersion of Comparative Example G and H correspond to Examples 8 and 9 of the co-pending International patent application PCT/US15/018390 having a filing date of 03 March 2015 and a first priority date of 08 April 2014.

Comparative Example I (not prior art) and Examples 1 and 2

To produce an aqueous HPMCAS dispersion, HPMCAS, surfactant and co-surfactant were added to water recirculating through a Drais DCP-12 Advantis media mill (1.0 mm ceramic media, 0.5 mm screen size). The mill speed was initially set at 1400-1500 rpm and then reduced as necessary down to about 1100-1400 rpm to control the mill outlet temperature.

HPMCAS and surfactant were pre-blended at a weight ratio to provide a percentage of surfactant based on HPMCAS, as listed in Table 1 below. The pre-blend of HPMCAS and surfactant was loaded in portions to water recirculating through the mill. Co-surfactant was loaded in portions throughout to provide a percentage of co-surfactant based on HPMCAS as listed in Table 1 below. The co-surfactant Aerosil OT 75 PG used in Examples 1 and 2 was a 75 wt.-% solution of dioctyl sodium sulfosuccinate in propylene glycol and water comprising 0.12 wt. carboxymethyl cellulose. Aerosil OT 75 PG is commercially available from Cytec Industries Inc. The weight percent co-surfactant, based on HPMCAS, as listed in Table 1 below refers to the total weight of Aerosil OT 75 PG. Addition continued until a total solids loading of 30-40% was achieved, based on the total weight of the composition. The percentage of HPMCAS, based on the total weight of the composition, was calculated from the measured solids content and the given weight ratio between HPMCAS, the surfactant and the optional co-surfactant. Following the addition of all solids, milling continued until the final particle size was obtained. The milling time was about 3 hours in Comparative Example I, about 3.5 hours in Example 1 and about 5 hours in Example 2. During the milling process, mild heat treatment was applied by adjusting the mill speed and the setpoint for the chiller system used to provide cooling to the mill jacket, tank and heat exchangers. The maximum temperature during milling was 39 - 41 °C.

Examples 3 and 4

To produce an aqueous HPMCAS dispersion, the same mill as in Examples 1 and 2 was used. The mill speed was initially set at 1300 - 1400 rpm and then reduced as necessary down to about 1100 rpm to control the mill outlet temperature.

HPMCAS and surfactant were pre-blended at a weight ratio to provide percentage of surfactant, based on HPMCAS, as listed in Table 1 below. The pre-blend of HPMCAS and surfactant was loaded in portions to water recirculating through the mill. Co-surfactant was loaded in portions throughout to provide a percentage of co-surfactant based on HPMCAS as listed in Table 1 below. The co-surfactant "DSS 50%" as listed in Table 1 below was a 50 wt.-% solution of dioctyl sodium sulfosuccinate in polyethylene glycol 400, NF. The weight percent co-surfactant, based on HPMCAS, as listed in Table 1 below refers to the total weight of the 50% dioctyl sodium sulfosuccinate solution. Addition continued and mild heat treatment was applied during the subsequent milling as described in Examples 1 and 2. The temperature during milling was most of the time from 30 to 40 °C, but during about 50 min. the temperature was more than 40 °C, the maximum being about 46 °C. The total milling time was about 2 hours in Example 3.

Example 4 was as a repetition of Example 3. The particle size distribution, the viscosity and the phase transition temperature of the freshly prepared dispersion of Example 4 was determined. The results of the analytical tests are listed in Table 1 below.

Examples 5 - 7

Example 3 was repeated except that a different heat treatment was applied during milling than in Example 3.

In Example 5 the temperature during milling was 43 - 47 °C for 2¾ hours, for the remaining time the milling temperature was 35- 40°C. The total milling time was about 4.25 h.

In Example 6 the temperature during milling was 43 - 47 °C for about 4 hours. The total milling time was about 4.25 hours.

Example 7 was as a repetition of Example 6. The particle size distribution, the viscosity and the phase transition temperature of the freshly prepared dispersion of Example 7 was determined. The results of the analytical tests are listed in Table 1 below.

Examples 8 - 10

To produce an aqueous HPMCAS dispersion, HPMCAS, surfactant and optionally ethylcellulose were preblended and added to water recirculating through a Drais DCP-12 Advantis media mill (1.0 mm ceramic media, 0.5 mm screen size). The mill speed was initially set at 1300-1450 rpm and then reduced as necessary down to about 1200-1400 rpm to control the mill outlet temperature. Co-surfactant was added periodically throughout the addition. The surfactant and co-surfactant were those as listed in Table 1 below.

In Examples 9 and 10, ethylcellulose was added that comprised 48.0 - 49.5 wt.% ethyl groups and had a viscosity of 9 - 11 mPa·s, measured as a 5% solution in 80% toluene and 20% ethanol at 25 °C in an Ubbelohde viscometer. The ethylcellulose is commercially available from The Dow Chemical Company as Ethocel Std. 10. It was used as a film forming aid.

Addition of HPMCAS, surfactant, co-surfactant and optionally ethylcellulose continued until a total solids loading of about 30 % was achieved, based on the total weight of the composition. The components were blended at weight ratios to provide percentages of surfactant and co-surfactant, based on polymer weight, as listed in Table 1 below. In Example 8, the polymer weight consisted of HPMCAS. In Example 9, the polymer weight consisted of a blend of 92 weight parts of HPMCAS and 5 weight parts of ethylcellulose. In Example 10, the polymer weight consisted of a blend of 86.4 weight parts of HPMCAS and 10.4 weight parts of ethylcellulose.

Table 1 below illustrates that the dispersions of Comparative Examples A, B and E showed agglomeration upon storage for about 2 weeks. Also the dispersion of Comparative Example D showed some agglomeration upon storage for about 2 weeks.

The dispersions of Comparative Examples F, G and H which are disclosed in co-pending patent application PCT/US15/018390, filed March 3, 2015, the dispersion of Comparative Example I and the dispersions of Examples 1 - 7 did not show agglomeration upon storage for about 2 weeks. The dispersion of Comparative Example I is not prior art.

The dispersions of Comparative Example I and of Examples 1- 3 that had undergone a mild heat treatment in the mill could be stored for more than 3 months without losing their ability to flow under their own weight. The dispersions of Examples 5 and 6 that had undergone heat treatment for an extended time period in the mill could even be stored for more than 6 months without losing their ability to flow under their own weight. The dispersions of Examples 4 and 7 were freshly prepared; therefore the stability test data is lacking.

Examples 1, 2, 6 and 7 illustrate that stable dispersions can be produced even at a very high HPMCAS concentrations of about 40 wt. %. Moreover, the dispersion of Example 7 has a viscosity in the same order of magnitude as that of Comparative Examples A, B and E, although the HPMCAS concentration in the dispersion of Example 7 is nearly twice as high as that in Comparative Examples A, B and E.

It was found that in the dispersions of Examples 1 - 7, which all comprise a co-surfactant as claimed, improved de-aeration was achieved, as compared to Comparative Example I which did not comprise a co-surfactant. Less air bubbles were formed and/or formed air bubbles could be more easily removed from the dispersions of the present invention. This is important for producing capsules and coatings of high transparency and good quality.

Fig. 1 - 4 illustrate the viscosity of the dispersions of Comparative Example I and of Examples 1, 2, 3, 6 and 5 as function of the shear rate at a temperature of 21 °C. Typically the viscosity of most fluids is independent or decreasing with increasing shear rates. Surprisingly the dispersions of the present invention, such as those of Examples 1, 2, 3, 5 and 6 show an increase of the viscosity with increasing shear rate. Fig. 1 - 4 illustrate a linear plot of the viscosity as a function of the shear rate in log scale. This viscosity increase is at least 20 mPa*s, typically at least 70 mPa*s, most typically at least 100 mPa*s above the previous lower shear rate and this viscosity increase is at least stable for two sequential shear rates (including 5 data points each decade).

Preparation of Films:

Example 11: The dispersion of Example 8 was stirred at 22 °C for 5 min. at 200 rpm. Then 18.8 % triethyl citrate (TEC), based on the weight of HPMCAS, was added drop wise to the dispersion. The TEC addition decreased the phase transition temperature of the dispersion. The resulting solids content of the dispersion was 33.9%. Stirring under moderate shear continued for additional 20 - 45 min at 22 °C. Then the film was cast on a non-sticking plate and dried at 55 - 75 °C for about an hour. The thickness of the produced film was about 157 micrometers.

Example 12: The dispersion of Example 10 was stirred at 22 °C for 5 min. at 200 rpm. Then a mixture of 24.2% of 34.8% tributyl citrate (TBC), 26.1% triethyl citrate (TEC), 34.8 % dibutyl sebacate (DBS) and 4.3 % of a poloxamer (plasticizer composition), based on the total weight of HPMCAS and ethyl cellulose, was added drop wise to the dispersion. The poloxamer was Pluronic L121, which is a poly(ethylene oxide) (PEO) - poly(propylene oxide) (PPO) - poly(ethylene oxide) (PEO) triblock copolymer of the structure $PEO_5\text{-}PPO_{68}\text{-}PEO_5$ and is commercially available from BASF Corporation. The resulting solids content of the dispersion was 35.4%. Then the film was cast on a non-sticking plate and dried as described in Example 11. The thickness of the produced film was about 152 micrometers.

Film Disintegration: The disintegration behavior of the films of Examples 11 and 12 was measured using a ball sample holder in conjunction with a disintegration tester QC-21 by Hanson Research (Chatsworth, CA). The instrument was equipped with a rigid basket-rack assembly supporting six cylindrical plastic tubes 77.5 mm long, 21.5 mm in internal diameter as described in the European Pharmacopeia (Seventh edition, 2011, Disintegration of Tablets and Capsules). The film was held between sample holders that was described in details in Pharmaceutical Technology May 1, 2012 and US2015/0150817. The film sample assembly with a steel ball placed on top of the film was then placed into the cylindrical plastic tube. The basket-rack assembly with six film assembly was first suspended in 0.1 N HCl buffer of a pH = 1.2 at 37±0.5 °C. The films of Examples 11 and 12 did not dissolve in 0.1 N HCl buffer. Then the films were immersed in a phosphate buffer of 0.2 M tribasic sodium phosphate that had been equilibrated to 37±0.5 °C and that had a pH of 6.8. The film of Example 11 dissolved in about 200 seconds. The film of Example 12 dissolved in about 300 seconds.

Table 1

| (Comparative) Example | HPMCAS based on total [%] | Surfactant | | Co-surfactant | | Mean (μm) | d50 (μm) | d90 (μm) | Viscosity at 20 °C [mPa*s] | Phase Transi-tion temp. [°C] | Stability after about 2 weeks | Mean after 2 weeks (μm) |
|---|---|---|---|---|---|---|---|---|---|---|---|---|
| | | type | wt.%, based on polymer 2) | type | wt.%, based on polymer 2) | | | | | | | |
| A | 20.4 | - | 0.0 | - | - | 3.6 | 3.2 | 6.7 | 5080 | < 15 | Aggl. | 44.8 |
| B | 20.0 | Tween 80 | 5.6 | - | - | 32.4 | 4.5 | 108.1 | 2890 | < 15 | Aggl. | 91.5 |
| C 1) | | SDS | 4.8 | - | - | NA | NA | NA | NA | NA | NA | NA |
| D | 24.2 | SDS | 0.7 | - | - | 1.9 | 1.4 | 3.9 | 112 | 19.0 | Aggl. | 6.5 |
| E | 20.8 | Pluronic L-44 | 4.8 | - | - | 1.9 | 1.4 | 3.9 | 3140 | <15 | Increased particle size | 57.9 |
| F* | 25.0 | Potassium Stearate | 5.0 | - | - | 2.4 | 1.2 | 4.7 | 130 | 35.4 | No Aggl. | 1.7 |
| G* | 28.6 | Sodium Stearate | 4.4 | - | - | 2.0 | 1.3 | 4.2 | 243 | 28.4 | No Aggl. | 1.6 |
| H* | 27.6 | Sodium Stearate | 3.8 | - | - | 2 | 1.4 | 4.4 | 108 | 34.6 | No Aggl. | 2.0 |
| I** | 28.5 | Sodium Stearate | 4.8 | - | - | 1.7 | 1.2 | 3.7 | NA | NA | No Aggl. | NA |
| 1 | 35.7 | Sodium Stearate | 3.0 | Aerosil OT 75 | 1.0 | 2.4 | 2.0 | 4.9 | NA | NA | No Aggl. | 2.5 |
| 2 | 39.5 | Potassium Stearate | 3.0 | Aerosil OT 75 | 0.07 | 3.0 | 2.6 | 5.5 | NA | NA | No Aggl. | 3.2 |
| 3 | 28.3 | Sodium Stearate | 3.0 | DSS 50% | 1.0 | 2.4 | 1.9 | 4.8 | NA | NA | No Aggl. | 2.8 |
| 4 | 28.3 | Sodium Stearate | 3.0 | DSS 50% | 1.0 | 2.4 | 2.0 | 4.9 | 115 | 40.3 | No Aggl. | 3.1 |
| 5 | 28.6 | Sodium Stearate | 4.0 | DSS 50% | 1.0 | 2.4 | 1.9 | 4.9 | NA | NA | No Aggl. | 2.4 |
| 6 | 38.1 | Sodium Stearate | 4.0 | 50% | 1.0 | 2.2 | 2.1 | 4.1 | NA | NA | No Aggl. | 2.9 |

(continued)

| (Comparative) Example | HPMCAS based on total [%] | Surfactant | | Co-surfactant | | Mean (μm) | d50 (μm) | d90 (μm) | Viscosity at 20 °C [mPa*s] | Phase Transi-tion temp. [°C] | Stability after about 2 weeks | Mean after 2 weeks (μm) |
|---|---|---|---|---|---|---|---|---|---|---|---|---|
| | | type | wt.%, based on polymer 2) | type | wt.%, based on polymer 2) | | | | | | | |
| 7 | 37.8 | Sodium Stearate | 4.0 | DSS 50% | 1.0 | 2.0 | 1.7 | 3.8 | 3640 | 24 | No Aggl. | 2.3 |
| 8 | 29.2 | Sodium Stearate | 3.0 | Aerosil OT 75 | 1.0 | 2.3 | 2.0 | 4.7 | NA | NA | No Aggl | NA |
| 9 | 29.6 | Sodium Stearate | 3.0 | Aerosil OT 75 | 0.07 | 2.3 | 1.8 | 4.6 | NA | NA | No Aggl | NA |
| 10 | 30.1 | Sodium Stearate | 3.2 | Aerosil OT 75 | 0.2 | 2.2 | 1.8 | 4.3 | NA | NA | No Aggl | NA |

NA: not assessed Aggl: Agglomeration No Aggl: No Agglomeration

[1] viscosity too high, the mill was inoperable

[2] polymer weight was HPMCAS weight, except in Examples 9 and 10, where polymer weight was total of HPMCAS and ethylcellulose * disclosed in co-pending application PCT/US15/018390, filed March 3, 2015 ; ** Comparative, but not prior art

**Claims**

1.  An aqueous composition comprising

    a) at least 25 percent, based on the total weight of the aqueous composition, of a dispersed esterified cellulose ether comprising (i) groups of the formula -C(O)-R-COOA or (ii) a combination of aliphatic monovalent acyl groups and groups of the formula -C(O)-R-COOA, wherein R is a divalent aliphatic or aromatic hydrocarbon group and A is hydrogen or a cation,
    b) from 0.1 to 15 percent of a salt of a fatty acid, based on the weight of the dispersed esterified cellulose ether, and
    c) from 0.02 to 7 percent of an anionic surfactant comprising a sulfate or sulfonate group, based on the weight of the dispersed esterified cellulose ether.

2.  The aqueous composition of claim 1 comprising from 0.5 to 10 percent of an ammonium, alkali metal or alkaline earth metal salt of a saturated or unsaturated fatty acid, based on the weight of the dispersed esterified cellulose ether.

3.  The aqueous composition of claim 1 or claim 2 wherein the salt of a fatty acid is an ammonium, alkali metal or alkaline earth metal salt of stearic acid or oleic acid.

4.  The aqueous composition of any one of claims 1 to 3 comprising from 0.05 to 5 percent of an anionic surfactant comprising a sulfonate group, based on the weight of the dispersed esterified cellulose ether.

5.  The aqueous composition of any one of claims 1 to 4 wherein the anionic surfactant comprising a sulfate or sulfonate group is a dialkyl sulfosuccinate.

6.  The aqueous composition of any one of claims 1 to 5, wherein the median particle size, d50, of the dispersed esterified cellulose ether particles is up to 7 micrometers, the median particle size, d50, being the size at which 50 volume percent of the particles have a smaller equivalent diameter and 50 volume percent have a larger equivalent diameter.

7.  The aqueous composition of any one of claims 1 to 6 comprising at least 30 weight percent of said at least one dispersed esterified cellulose ether, based on the total weight of the aqueous composition.

8.  The aqueous composition of any one of claims 1 to 7 wherein the aliphatic monovalent acyl groups in the esterified cellulose ether are acetyl, propionyl or butyryl groups, and the groups of the formula -C(O)-R-COOA are -C(O)-CH$_2$-CH$_2$-COOA, -C(O)-CH=CH-COOA, or -C(O)-C$_6$H$_4$-COOA groups.

9.  The aqueous composition of any one of claims 1 to 8 wherein the esterified cellulose ether is hydroxypropyl methyl cellulose acetate succinate.

10. A process for producing the aqueous composition of any one of claims 1 to 9 comprising the steps of
    grinding, in the presence of an aqueous diluent, an esterified cellulose ether comprising (i) groups of the formula -C(O)-R-COOA or (ii) a combination of aliphatic monovalent acyl groups and groups of the formula -C(O)-R-COOA, wherein R is a divalent aliphatic or aromatic hydrocarbon group and A is hydrogen or a cation,
    blending a salt of a fatty acid, an anionic surfactant comprising a sulfate or sulfonate group and optionally one or more adjuvants with the esterified cellulose ether before, during or after the grinding of the esterified cellulose ether, and
    choosing the amounts of aqueous diluent, esterified cellulose ether, salt of a fatty acid, anionic surfactant comprising a sulfate or sulfonate group and optionally one or more adjuvants such that the produced aqueous composition comprises a) at least 25 percent, based on the total weight of the aqueous composition, of the dispersed esterified cellulose ether, b) from 0.1 to 15 percent of the salt of a fatty acid, and c) from 0.02 to 7 percent of the anionic surfactant comprising a sulfate or sulfonate group, the percentages of components b) and c) being based on the weight of the dispersed esterified cellulose ether.

11. The process of claim 10 wherein the esterified cellulose ether, the salt of a fatty acid and optionally the anionic surfactant comprising a sulfate or sulfonate group are ground together in the presence of an aqueous diluent at a temperature of from 37 to 55 °C.

12. A process for producing the aqueous composition of any one of claims 1 to 9 comprising the steps of

melting an esterified cellulose ether comprising (i) groups of the formula -C(O)-R-COOA or (ii) a combination of aliphatic monovalent acyl groups and groups of the formula -C(O)-R-COOA, wherein R is a divalent aliphatic or aromatic hydrocarbon group and A is hydrogen or a cation, and

emulsifying the molten esterified cellulose ether in an aqueous diluent,

adding a salt of a fatty acid and an anionic surfactant comprising a sulfate or sulfonate group and optionally one or more adjuvants before, during or after the step of emulsifying the molten esterified cellulose ether in the aqueous diluent,

choosing the amounts of aqueous diluent, esterified cellulose ether, salt of a fatty acid, anionic surfactant comprising a sulfate or sulfonate group and optionally one or more adjuvants such that the produced aqueous composition comprises a) at least 25 percent, based on the total weight of the aqueous composition, of the dispersed esterified cellulose ether, b) from 0.1 to 15 percent of the salt of a fatty acid, and c) from 0.02 to 7 percent of the anionic surfactant comprising a sulfate or sulfonate group, the percentages of components b) and c) being based on the weight of the dispersed esterified cellulose ether,

and cooling the emulsion to form an aqueous dispersion.

13. A dosage form being coated with a coating prepared from the aqueous composition of any one of claims 1 to 9.

14. A capsule shell made from the aqueous composition of any one of claims 1 to 9.

15. A process for producing capsule shells comprising the steps of providing the aqueous composition of any one of claims 1 to 9,

pre-heating molding pins to a temperature higher than the aqueous composition,

dipping the pre-heated molding pins into the aqueous composition,

forming a film on said molding pins by withdrawing said pins from said aqueous composition, and

drying the film on the molding pins.

**Patentansprüche**

1. Wässrige Zusammensetzung enthaltend:

   a) wenigstens 25 Prozent, bezogen auf das Gesamtgewicht der wässrigen Zusammensetzung, eines dispergierten veresterten Celluloseethers, der (i) Gruppen der Formel -C(O)-R-COOA oder (ii) eine Kombination von aliphatischen monovalenten Acylgruppen und Gruppen der Formel -C(O)-R-COOA enthält, worin R eine divalente aliphatische oder aromatische Kohlenwasserstoffgruppe ist und A Wasserstoff oder ein Kation ist,
   b) 0,1 bis 15 Prozent eines Salzes einer Fettsäure, bezogen auf das Gewicht des dispergierten veresterten Celluloseethers, und
   c) 0,02 bis 7 Prozent eines anionischen oberflächenaktiven Mittels, das eine Sulfat- oder Sulfonatgruppe enthält, bezogen auf das Gewicht des dispergierten veresterten Celluloseethers.

2. Wässrige Zusammensetzung nach Anspruch 1, die 0,5 bis 10 Prozent eines Ammonium-, Alkalimetall- oder Erdalkalimetallsalzes einer gesättigten oder ungesättigten Fettsäure, bezogen auf das Gewicht des dispergierten veresterten Celluloseethers, enthält.

3. Wässrige Zusammensetzung nach Anspruch 1 oder Anspruch 2, wobei das Salz einer Fettsäure ein Ammonium-, Alkalimetall- oder Erdalkalimetallsalz von Stearinsäure oder Ölsäure ist.

4. Wässrige Zusammensetzung nach einem der Ansprüche 1 bis 3, die 0,05 bis 5 Gewichtsprozent eines anionischen oberflächenaktiven Mittels, das Sulfonatgruppen enthält, bezogen auf das Gewicht des dispergierten veresterten Celluloseethers, enthält.

5. Wässrige Zusammensetzung nach einem der Ansprüche 1 bis 4, wobei das anionische oberflächenaktive Mittel, das eine Sulfat- oder Sulfonatgruppe enthält, ein Dialkylsulfosuccinat ist.

6. Wässrige Zusammensetzung nach einem der Ansprüche 1 bis 5, wobei die Medianteilchengröße d50 des dispergierten veresterten Celluloseethers bis zu 7 Micrometer beträgt, wobei die Medianteilchengröße d50 die Größe ist, bei der 50 Volumenprozent der Teilchen einen kleineren äquivalenten Durchmesser und 50 Volumenprozent einen größeren äquivalenten Durchmesser aufweisen.

7. Wässrige Zusammensetzung nach einem der Ansprüche 1 bis 6, die wenigstens 30 Gewichtsprozent dieses wenigstens einen dispergierten veresterten Celluloseethers, bezogen auf das Gesamtgewicht der wässrigen Zusammensetzung, enthält.

8. Wässrige Zusammensetzung nach einem der Ansprüche 1 bis 7, wobei die aliphatischen monovalenten Acylgruppen in dem veresterten Celluloseether Acetyl-, Propionyl- oder Butyrylgruppen sind und die Gruppen der Formel -C(O)-R-COOA die Gruppen -C(O)-$CH_2$-$CH_2$-COOA, -C(O)-CH=CH-COOA oder -C(O)-$C_6H_4$-COOA Gruppen.

9. Wässrige Zusammensetzung nach einem der Ansprüche 1 bis 8, wobei der veresterte Celluloseether Hydroxypropylmethylcelluloseacetatsuccinat ist.

10. Verfahren zur Herstellung der wässrigen Zusammensetzung nach einem der Ansprüche 1 bis 9, umfassend die Schritte:

Mahlen, in Gegenwart eines wässrigen Verdünnungsmittels, eines veresterten Celluloseethers, der (i) Gruppen der Formel -C(O)-R-COOA oder (ii) eine Kombination von aliphatischen monovalenten Acylgruppen und Gruppen der Formel -C(O)-R-COOA enthält, worin R eine divalente aliphatische oder aromatische Kohlenwasserstoffgruppe ist und A Wasserstoff oder ein Kation ist,
Mischen eines Salzes einer Fettsäure, eines anionischen oberflächenaktiven Mittels, das eine Sulfat- oder Sulfonatgruppe enthält, und optional eines oder mehrerer Hilfsmittel mit dem veresterten Celluloseether vor, während oder nach dem Mahlen des veresterten Celluloseethers und Auswählen der Mengen des wässrigem Verdünnungsmittels, des veresterten Celluloseethers, des Salzes einer Fettsäure, des anionischen oberflächenaktiven Mittels, das eine Sulfat- oder Sulfonatgruppe enthält, und optional eines oder mehrerer Hilfsmittel, sodass die hergestellte wässrige Zusammensetzung a) wenigstens 25 Gewichtsprozent, bezogen auf das Gesamtgewicht der wässrigen Zusammensetzung, des dispergierten veresterten Celluloseethers, b) 0,1 bis 15 Gewichtsprozent des Salzes einer Fettsäure und c) 0,02 bis 7 Gewichtsprozent des anionischen oberflächenaktiven Mittels, das eine Sulfat- oder Sulfonatgruppe enthält, enthält, wobei die Prozentangaben der Komponenten b) und c) sich auf das Gewicht des dispergierten veresterten Celluloseethers beziehen.

11. Verfahren nach Anspruch 10, wobei der veresterte Celluloseether, das Salz einer Fettsäure und optional das anionische oberflächenaktive Mittel, das eine Sulfat- oder Sulfonatgruppe enthält, zusammen in Gegenwart eines wässrigen Verdünnungsmittels bei einer Temperatur von 37 bis 55 °C gemahlen werden.

12. Verfahren zur Herstellung der wässrigen Zusammensetzung nach einem der Ansprüche 1 bis 9, umfassend die Schritte:

Schmelzen eines veresterten Celluloseethers, der (i) Gruppen der Formel -C(O)-R-COOA oder (ii) eine Kombination von aliphatischen monovalenten Acylgruppen und Gruppen der Formel -C(O)-R-COOA enthält, worin R eine divalente aliphatische oder aromatische Kohlenwasserstoffgruppe ist und A Wasserstoff oder ein Kation ist, und
Emulgieren des geschmolzenen veresterten Celluloseethers in einem wässrigen Verdünnungsmittel,
Zugeben eines Salzes einer Fettsäure und eines anionischen oberflächenaktiven Mittels, das eine Sulfat- oder Sulfonatgruppe enthält, und optional eines oder mehrerer Hilfsstoffe vor, während oder nach dem Schritt des Emulgierens des geschmolzenen veresterten Celluloseethers in dem wässrigen Verdünnungsmittel,
Auswählen der Mengen an wässrigem Verdünnungsmittel, verestertem Celluloseether, Salz einer Fettsäure, anionischem oberflächenaktiven Mittels, das eine Sulfat- oder Sulfonatgruppe enthält, und optional von einem oder mehreren Hilfsmitteln, sodass die hergestellte wässrige Zusammensetzung a) wenigstens 25 Prozent, bezogen auf das Gesamtgewicht der wässrigen Zusammensetzung, des dispergierten veresterten Celluloseethers, b) 0,1 bis 15 Prozent des Salzes einer Fettsäure und c) 0,02 bis 7 Prozent des anionischen oberflächenaktiven Mittels, das eine Sulfat- oder Sulfonatgruppe enthält, enthält, wobei die Prozentangaben der Komponenten b) und c) sich auf das Gesamtgewicht des dispergierten veresterten Celluloseethers beziehen, und Abkühlen der Emulsion, um eine wässrige Dispersion zu bilden.

13. Dosierform, beschichtet mit einer Beschichtung, hergestellt aus der wässrigen Zusammensetzung nach einem der Ansprüche 1 bis 9.

14. Kapselschale, hergestellt aus der wässrigen Zusammensetzung nach einem der Ansprüche 1 bis 9.

**15.** Verfahren zur Herstellung von Kapselschalen, umfassend die Schritte:

Bereitstellen der wässrigen Zusammensetzung nach einem der Ansprüche 1 bis 9,
Vorerwärmen der Formstifte auf eine Temperatur, die höher ist als die der wässrigen Zusammensetzung,
Eintauchen der vorgewärmten Formstifte in die wässrige Zusammensetzung,
Ausbilden eines Films auf diesen Formstiften durch Herausziehen der Stifte aus dieser wässrigen Zusammensetzung und Trocknen des Films auf den Formstiften.

**Revendications**

**1.** Une composition aqueuse comprenant

a) au moins 25 pour cent, rapporté au poids total de la composition aqueuse, d'un éther de cellulose estérifié dispersé comprenant (i) des groupes de la formule -C(O)-R-COOA ou (ii) une combinaison de groupes acyle monovalents aliphatiques et de groupes de la formule -C(O)-R-COOA, dans laquelle R est un groupe hydrocarbure aliphatique ou aromatique divalent et A est un hydrogène ou un cation,
b) de 0,1 à 15 pour cent d'un sel d'un acide gras, rapporté au poids de l'éther de cellulose estérifié dispersé, et
c) de 0,02 à 7 pour cent d'un agent tensioactif anionique comprenant un groupe sulfate ou sulfonate, rapporté au poids de l'éther de cellulose estérifié dispersé.

**2.** La composition aqueuse de la revendication 1 comprenant de 0,5 à 10 pour cent d'un sel d'ammonium, de métal alcalin ou de métal alcalino-terreux d'un acide gras saturé ou insaturé, rapporté au poids de l'éther de cellulose estérifié dispersé.

**3.** La composition aqueuse de la revendication 1 ou de la revendication 2 dans laquelle le sel d'un acide gras est un sel d'ammonium, de métal alcalin ou de métal alcalino-terreux d'acide stéarique ou d'acide oléique.

**4.** La composition aqueuse de l'une quelconque des revendications 1 à 3 comprenant de 0,05 à 5 pour cent d'un agent tensioactif anionique comprenant un groupe sulfonate, rapporté au poids de l'éther de cellulose estérifié dispersé.

**5.** La composition aqueuse de l'une quelconque des revendications 1 à 4 dans laquelle l'agent tensioactif anionique comprenant un groupe sulfate ou sulfonate est un sulfosuccinate de dialkyle.

**6.** La composition aqueuse de l'une quelconque des revendications 1 à 5, dans laquelle la taille de particule médiane, d50, des particules d'éther de cellulose estérifié dispersé va jusqu'à 7 micromètres, la taille de particule médiane, d50, étant la taille à laquelle 50 pour cent en volume des particules ont un diamètre équivalent plus petit et 50 pour cent en volume ont un diamètre équivalent plus grand.

**7.** La composition aqueuse de l'une quelconque des revendications 1 à 6 comprenant au moins 30 pour cent en poids dudit au moins un éther de cellulose estérifié dispersé, rapporté au poids total de la composition aqueuse.

**8.** La composition aqueuse de l'une quelconque des revendications 1 à 7 dans laquelle les groupes acyle monovalents aliphatiques dans l'éther de cellulose estérifié sont des groupes acétyle, propionyle ou butyryle, et les groupes de la formule -C(O)-R-COOA sont des groupes -C(O)-$CH_2$-$CH_2$-COOA, -C(O)-CH=CH-COOA, ou -C(O)-$C_6H_4$-COOA.

**9.** La composition aqueuse de l'une quelconque des revendications 1 à 8 dans laquelle l'éther de cellulose estérifié est l'acétate succinate d'hydroxypropylméthylcellulose.

**10.** Un procédé pour produire la composition aqueuse de l'une quelconque des revendications 1 à 9 comprenant les étapes
de broyage, en présence d'un diluant aqueux, d'un éther de cellulose estérifié comprenant (i) des groupes de la formule -C(O)-R-COOA ou (ii) une combinaison de groupes acyle monovalents aliphatiques et de groupes de la formule -C(O)-R-COOA, dans laquelle R est un groupe hydrocarbure aliphatique ou aromatique divalent et A est un hydrogène ou un cation,
de mélange homogène d'un sel d'un acide gras, d'un agent tensioactif anionique comprenant un groupe sulfate ou sulfonate et facultativement d'un ou de plusieurs adjuvants avec l'éther de cellulose estérifié avant, durant ou après le broyage de l'éther de cellulose estérifié, et

de choix des quantités de diluant aqueux, d'éther de cellulose estérifié, de sel d'un acide gras, d'agent tensioactif anionique comprenant un groupe sulfate ou sulfonate et facultativement d'un ou de plusieurs adjuvants de telle sorte que la composition aqueuse produite comprend a) au moins 25 pour cent, rapporté au poids total de la composition aqueuse, de l'éther de cellulose estérifié dispersé, b) de 0,1 à 15 pour cent du sel d'un acide gras, et c) de 0,02 à 7 pour cent de l'agent tensioactif anionique comprenant un groupe sulfate ou sulfonate, les pourcentages des constituants b) et c) étant rapportés au poids de l'éther de cellulose estérifié dispersé.

11. Le procédé de la revendication 10 dans lequel l'éther de cellulose estérifié, le sel d'un acide gras et facultativement l'agent tensioactif anionique comprenant un groupe sulfate ou sulfonate sont broyés ensemble en présence d'un diluant aqueux à une température allant de 37 à 55 °C.

12. Un procédé pour produire la composition aqueuse de l'une quelconque des revendications 1 à 9 comprenant les étapes de fusion d'un éther de cellulose estérifié comprenant (i) des groupes de la formule -C(O)-R-COOA ou (ii) une combinaison de groupes acyle monovalents aliphatiques et de groupes de la formule -C(O)-R-COOA, dans laquelle R est un groupe hydrocarbure aliphatique ou aromatique divalent et A est un hydrogène ou un cation, et d'émulsification de l'éther de cellulose estérifié fondu dans un diluant aqueux,
d'ajout d'un sel d'un acide gras et d'un agent tensioactif anionique comprenant un groupe sulfate ou sulfonate et facultativement d'un ou de plusieurs adjuvants avant, durant ou après l'étape d'émulsification de l'éther de cellulose estérifié fondu dans le diluant aqueux,
de choix des quantités de diluant aqueux, d'éther de cellulose estérifié, de sel d'un acide gras, d'agent tensioactif anionique comprenant un groupe sulfate ou sulfonate et facultativement d'un ou de plusieurs adjuvants de telle sorte que la composition aqueuse produite comprend a) au moins 25 pour cent, rapporté au poids total de la composition aqueuse, de l'éther de cellulose estérifié dispersé, b) de 0,1 à 15 pour cent du sel d'un acide gras, et c) de 0,02 à 7 pour cent de l'agent tensioactif anionique comprenant un groupe sulfate ou sulfonate, les pourcentages des constituants b) et c) étant rapportés au poids de l'éther de cellulose estérifié dispersé,
et refroidir l'émulsion afin de former une dispersion aqueuse.

13. Une forme galénique qui est enrobée d'un enrobage préparé à partir de la composition aqueuse de l'une quelconque des revendications 1 à 9.

14. Une enveloppe de gélule réalisée à partir de la composition aqueuse de l'une quelconque des revendications 1 à 9.

15. Un procédé pour produire des enveloppes de gélules comprenant les étapes de fourniture de la composition aqueuse de l'une quelconque des revendications 1 à 9, de préchauffage de broches de moulage jusqu'à une température supérieure à la composition aqueuse,
d'immersion des broches de moulage préchauffées dans la composition aqueuse,
de formation d'un film sur lesdites broches de moulage par retrait desdites broches de ladite composition aqueuse, et de séchage du film sur les broches de moulage.

Fig. 1

Fig. 2

Fig. 3

Fig. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4226981 A
- US 4365060 A
- US 7138143 B
- EP 0662323 A
- EP 0677322 A
- JP 8109124 A
- JP 7070203 A

- EP 0648487 A
- WO 2013164122 A
- US 5539021 A
- US 7763676 B
- WO 2013164121 A
- US 15018390 W
- US 20150150817 A

**Non-patent literature cited in the description**

- Hypromellose Acetate Succinate. *United States Pharmacopeia and National Formulary, NF,* vol. 29, 1548-1550
- Hypromellose. United States Pharmacopeia and National Formulary, USP, vol. 35, 3467-3469

- Hypromellose Acetate Succinate. United States Pharmacopia and National Formulary, NF, vol. 29, 1548-1550
- Disintegration of Tablets and Capsules. European Pharmacopeia, 2011